(19) Europäisches Patentamt
European Patent Office
Office européen des brevets

(11) **EP 3 384 847 B1**

(12) **EUROPÄISCHE PATENTSCHRIFT**

(45) Veröffentlichungstag und Bekanntmachung des Hinweises auf die Patenterteilung:
**12.06.2019 Patentblatt 2019/24**

(51) Int Cl.:
*A61B 6/00* (2006.01)   *A61B 6/03* (2006.01)

(21) Anmeldenummer: **17164992.4**

(22) Anmeldetag: **05.04.2017**

(54) **BESTIMMEN EINES REFERENZDOSISPARAMETERS EINER COMPUTERTOMOGRAPHIEBILDGEBUNG**

DETERMINING A REFERENCE DOSE PARAMETER OF COMPUTED TOMOGRAPHY IMAGING

DÉTERMINATION D'UN PARAMÈTRE DE DOSAGE DE RÉFÉRENCE D'UN SYSTÈME DE PRODUCTION D'IMAGE TOMOGRAPHIQUE PAR ORDINATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**10.10.2018 Patentblatt 2018/41**

(73) Patentinhaber: **Siemens Healthcare GmbH 91052 Erlangen (DE)**

(72) Erfinder: **Dorn, Karlheinz 90562 Kalchreuth (DE)**

(56) Entgegenhaltungen:
**WO-A1-2013/049818     WO-A1-2013/103790 US-A1- 2016 063 686**

• **J. MENKE: "Comparison of Different Body Size Parameters for Individual Dose Adaptation in Body CT of Adults", RADIOLOGY, Bd. 236, Nr. 2, 1. August 2005 (2005-08-01), Seiten 565-571, XP055075512, ISSN: 0033-8419, DOI: 10.1148/radiol.2362041327**

**Beschreibung**

In der Computertomographiebildgebung ist es neben einer guten Bildqualität auch das Ziel, dass das jeweilige Untersuchungsvolumen eine möglichst geringe Röntgendosis absorbiert, da eine hohe Röntgendosis kann zu einer potentiellen Schädigung von Teilen des Untersuchungsvolumens führen kann. Dieses Ziel wird auch als ALARA (englisches Akronym für "as low as reasonable achievable", eine deutsche Übersetzung ist "so niedrig wie vernünftigerweise erreichbar") bezeichnet.

Dabei sinkt im Allgemeinen bei einer steigenden Röntgendosis das Rauschen im resultierenden Bilddatensatz, wodurch die Bildqualität steigt. Eine Computertomographiebildgebung kann also als optimal bezeichnet werden, wenn die Bildqualität gerade gut genug gewählt wird, und damit wenn die Röntgendosis gerade hoch genug gewählt wird, um noch eine eindeutige Befundung des resultierenden Bildes zu ermöglichen. Es ist daher üblich, zur Planung und/oder zur Bewertung einer Computertomograhpiebildgebung Dosisparameter heranzuziehen.

Es ist bekannt, einen Dosisparameter bezüglich einer axialen Schnittebene des Untersuchungsvolumens basierend auf einem axialen Schnittbild und weiteren Aufnahmeparametern zu berechnen, beispielsweise mittels einer Monte-Carlo-Simulation. Hierbei kann die Röntgendosis aber nur nach der Computertomographiebildgebung berechnet werden, damit ist hier die Röntgendosis nur ein Ergebnis der Computertomographiebildgebung. Weiterhin müssen bei dieser Methode sehr viele Schnittbilder übertragen und analysiert werden, wenn die Röntgendosis in einem größeren Bereich des Untersuchungsvolumens bestimmt werden soll, weiterhin muss für diese Berechnung viel Rechenzeit aufgewendet werden.

Es ist weiterhin bekannt, einen Dosisparameter alleine basierend auf den Aufnahmeparametern einer Computertomographiebildgebung zu berechnen, wie z.B. in der WO-2013/103790 offenbart, bei der eine Referenzdosis auf Basis der Röhrenspannung berechnet wird. Diese Berechnung erfolgt spezifisch nur für einen bestimmten Hersteller von Computertomographiegeräten oder noch spezifischer für nur einen Typ von Computertomographiegeräten. Dadurch kann der jeweils berechnete Dosisparameter nur mit Einschränkungen zwischen Computertomographiegeräten unterschiedlicher Hersteller oder unterschiedlichen Typs verglichen werden. Weiterhin ist der jeweils berechnete Dosisparameter nicht individuell auf den Patienten abgestimmt.

Die Nachteile der großen Datenmenge und der schlechten Vergleichbarkeit zeigen sich vor allem, wenn geräteübergreifende oder krankenhausübergreifende Auswertungen des Dosisparameters mittels einer Umgebung für verteiltes Rechnen (ein englischer Fachbegriff ist "Cloud") durchgeführt werden sollen, wobei Bilddaten und/oder Untersuchungsdaten räumlich getrennt von einer Auswerteeinheit gespeichert werden und daher eine Datenübertragung stattfinden muss. Eine typische Aufgabenstellung einer solchen Auswertung ist, die bei einer bestimmten Computertomograhpiebildgebung (beispielsweise des Kopfes) vom Patienten absorbierte Röntgendosis in einem Krankenhaus mit dem Landesdurchschnitt zu vergleichen. Hierfür müssen Dosisparameter für verschiedene Gerätetypen aus Daten berechnet werden, die räumlich getrennt von der Auswerteeinheit gespeichert sind. Weiterhin müssen die notwendigen Daten auch für die Auswerteeinheit zugänglich sein.

Es ist daher die Aufgabe der vorliegenden Erfindung, eine Methode bereitzustellen, die Röntgendosis einer Computertomographiebildgebung unabhängig vom Ergebnis der Computertomographiebildgebung vergleichbar, schnell und ohne die Übertragung großer Datenmengen zu berechnen.

Die Aufgabe wird gelöst durch ein Verfahren zur Bestimmung eines Referenzdosisparameters einer Computertomographiebildgebung nach Anspruch 1, durch eine Parameterbestimmungseinheit nach Anspruch 11, durch ein Computertomographiegerät nach Anspruch 13, durch ein Computerprogrammprodukt nach Anspruch 14 sowie durch ein computerlesbares Speichermedium nach Anspruch 15.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Das erfindungsgemäße Verfahren zum Bestimmen eines Referenzdosisparameters einer Computertomographiebildgebung basiert darauf, dass ein Wasseräquivalenzdurchmessers (kurz "WED") einer Schnittebene eines Untersuchungsvolumens mittels einer Schnittstelle empfangen wird. Weiterhin wird ein Rauschpegel basierend auf dem Wasseräquivalenzdurchmesser mittels einer Recheneinheit bestimmt, wobei der Rauschpegel ein oberer Schwellenwert für das Rauschen eines CT-Bilddatensatzes ist. Weiterhin wird ein Referenzdosisparameters basierend auf dem Rauschpegel und dem Wasseräquivalenzdurchmesser mittels der Recheneinheit bestimmt, wobei der Referenzdosisparameter einer ersten Röntgendosis entspricht, welche bei einer Aufnahme eines ersten CT-Bilddatensatzes des Untersuchungsvolumens in der Schnittebene absorbiert werden würde, wenn das Rauschen des ersten CT-Bilddatensatzes dem Rauschpegel entspricht, und wobei der Referenzdosisparameter indirekt proportional zu einer Potenz des Rauschpegels ist. Mit anderen Worten ist es bei diesem Bestimmen nicht notwendig, den ersten CT-Bilddatensatz tatsächlich aufzunehmen, die erste Röntgendosis wird nur theoretisch in der Schnittebene absorbiert, wenn unter Verwendung des Aufnahmeparameters ein erstes CT-Bilddatensatz Untersuchungsvolumens mittels des Computertomographiegeräts aufgenommen werden würde.

Der Erfinder hat erkannt, dass der Rauschpegel als ein oberer Schwellenwert für das Rauschen eines CT-Bilddatensatzes besonders effizient und schnell basierend auf dem Wasseräquivalenzdurchmesser bestimmt werden kann. Weiterhin hat der Erfinder erkannt, dass sowohl der Wasseräquivalenzdurchmesser als auch der Rauschpegel dazu verwendet werden können, den Referenzdosisparameter besonders effizient zu bestimmen, da mit der vorgeschlagenen Methode keine großen Bilddatenmengen übertragen werden müssen, gleichzeitig aber auch Einflüsse der Beschaffenheit des Untersuchungsbereichs in die Bestimmung einfließen zu lassen. Insbesondere spiegelt der Wasseräquivalenzdurchmesser des Untersuchungsvolumens (beispielsweise eines Patienten) die physischen Gegebenheiten des Untersuchungsvolumens bezüglich der Schnittebene wieder, insbesondere besser als bei der Berechnung des Referenzdosisparameters basierend auf statistischen, nicht auf den jeweiligen Untersuchungsbereich und/oder den jeweiligen Patienten bezogen Daten. Der Erfinder hat weiterhin erkannt, dass der Referenzdosisparameter unabhängig vom Hersteller eines Computertomographiegeräts indirekt proportional zu einer Potenz des Rauschpegels ist, daher kann der Referenzdosisparameter insbesondere ohne Verwendung eines axialen Schnittbildes und sowie herstellerunabhängig bestimmt werden.

Nach einem weiteren Aspekt der Erfindung weist die Potenz einen Wert zwischen 0.1 und 2.5, insbesondere zwischen 0.5 und 1.5, insbesondere zwischen 0.9 und 1.1 auf. Insbesondere kann die Potenz einen Wert von genau 1 aufweisen. Der Erfinder hat erkannt, dass durch die Wahl eines derartigen Wertes der Referenzdosisparameter besonders genau bestimmt werden kann.

Nach einem weiteren Aspekt der Erfindung ist der Referenzdosisparameter weiterhin proportional zu einer Exponentialfunktion des Wasseräquivalenzdurchmessers. Der Erfinder hat erkannt, dass die funktionale Abhängigkeit des Referenzdosisparameters vom Wasseräquivalenzdurchmesser besonders gut und genau durch eine Exponentialfunktion beschrieben werden kann.

Nach einem weiteren Aspekt der Erfindung umfasst das Argument der Exponentialfunktion das Produkt des linearen Abschwächungskoeffizienten von Röntgenstrahlung in Wasser mit dem Wasseräquivalenzdurchmesser. Der Erfinder hat erkannt, dass auch der lineare Abschwächungskoeffizient von Wasser einen Einfluss auf die Größe des Referenzdosisparameters hat, und sich die gemeinsame funktionale Abhängigkeit des Referenzdosisparameters vom linearen Abschwächungskoeffizienten und vom Wasseräquivalenzdurchmesser besonders gut und genau durch eine Exponentialfunktion des Produkts der beiden Größen beschreiben lässt. Hierbei bezeichnet das Argument einer Funktion den Wert aus der Definitionsmenge der Funktion, der in die Funktion eingesetzt wird, um den zugehörigen Funktionswert zu bestimmen. Ein anderes Wort für Argument einer Funktion ist unabhängige Variable der Funktion. Der lineare Abschwächungskoeffizient von Röntgenstrahlung in Wasser ist insbesondere eine Länge, so dass die Intensität von Röntgenstrahlung auf einen Anteil von 1/e (1/e ≈ 0,368) der ursprünglichen Intensität absinkt, wenn die Röntgenstrahlung auf einer Strecke mit dieser Länge Wasser durchläuft, wobei e (e ≈ 2,718) hier die Eulerzahl bezeichnet. Insbesondere handelt es sich bei der Exponentialfunktion um eine Exponentialfunktion zur Basis der Eulerzahl e. Als linearer Abschwächungskoeffizient von Röntgenstrahlung in Wasser ist insbesondere 0,19 cm$^{-1}$ bekannt. Als Synonym für linearen Abschwächungskoeffizient ist auch Absorptionskoeffizient bekannt.

Nach einem weiteren Aspekt der Erfindung ist der Referenzdosisparameter das Produkt einer Proportionalitätskonstante mit dem Quotienten der Exponentialfunktion und der Potenz des Rauschpegels, wobei p der Wert der Potenz ist, wobei die Proportionalitätskonstante zwischen 0,2 mGy·HU$^p$ und 2,0 mGy·HU$^p$ beträgt, oder wobei die Proportionalitätskonstante insbesondere zwischen 0,5 mGy·HU$^p$ und 1,5 mGy·HU$^p$ beträgt, oder wobei die Proportionalitätskonstante insbesondere zwischen 1,2 mGy·HU$^p$ und 1,2 mGy·HU$^p$ beträgt. Hierbei bezeichnet die "mGy·HU$^p$" die Einheit Milligray mal mit p potenzierte Hounsfieldeinheit. Hierbei ist die Einheit ein Gray insbesondere als ein Joule pro Kilogramm definiert, und die Hounsfieldeinheit ist insbesondere die Einheit der CT-Zahl. Der Erfinder hat erkannt, dass durch die Wahl einer derartigen Proportionalitätskonstante der Referenzdosisparameter besonders gut und genau bestimmt werden kann.

Nach einem weiteren Aspekt der Erfindung ist der Rauschpegel eine stückweise lineare Funktion des Wasseräquivalenzdurchmessers. Der Erfinder hat erkannt, dass durch diese funktionale Abhängigkeit der Rauschpegel besonders gut in Abhängigkeit des Wasseräquivalenzdurchmessers bestimmt werden kann. Eine Funktion ist insbesondere dann stückweise linear, wenn die zweite Ableitung der Funktion an jeder Stelle entweder null oder nicht definiert ist.

Nach einem weiteren Aspekt der Erfindung ist der Rauschpegel eine stetige, abschnittsweise definierte Funktion des Wasseräquivalenzdurchmessers mit genau zwei Abschnitten, wobei der erste Abschnitt eine konstante Funktion ist und wobei der zweite Abschnitt eine lineare Funktion des Wasseräquivalenzdurchmessers ist. Der Erfinder hat erkannt, dass durch diese funktionale Abhängigkeit der Rauschpegel besonders gut in Abhängigkeit des Wasseräquivalenzdurchmessers bestimmt werden kann. Insbesondere umfasst der erste Abschnitt kleinere Wasseräquivalenzdurchmesser als der zweite Abschnitt.

Der Rauschpegel s kann insbesondere durch die Formel s = max(c, m·WED - t) gegeben sein, wobei die Größen c, m und t Konstanten sind. Die Konstante c kann insbesondere zwischen 0 HU und 10 HU, insbesondere zwischen 2 HU und 6 HU oder insbesondere zwischen 3 HU und 5 HU gewählt werden, weiterhin kann die Konstante c insbesondere als 4 HU gewählt werden. Die Konstante m kann insbesondere zwischen 0,5 HU/cm und 3 HU/cm, insbesondere zwischen 0,5 HU/cm und 2 HU/cm, insbesondere zwischen 0,75 HU/cm und 1,25 HU/cm gewählt werden, insbesondere kann die Konstante m auch als 1,0 HU/cm gewählt werden. Die Konstante t kann zwischen 0 HU und 30 HU, insbesondere zwischen 10 HU und 20 HU, insbesondere zwischen 12 HU und 16 HU gewählt werden, die Konstante t kann aber auch insbesondere als 14 HU gewählt werden. Der Erfinder hat erkannt, dass durch eine derartige Wahl einer oder mehrerer der Konstanten die Abhängigkeit des Rauschpegels vom Wasseräquivalenzdurchmesser besonders gut beschrieben werden kann.

Nach einem weiteren Aspekt der Erfindung wird weiterhin eine größenspezifischen Dosisschätzung (ein englischer Fachbegriff ist "size-specific dose estimation", kurz "SSDE") basierend auf dem Referenzdosisparameter mittels der Recheneinheit bestimmt. Insbesondere wird die größenspezifische Dosisschätzung als Produkt des Referenzdosisparameters mit einer Funktion berechnet, wobei die Funktion vom Wasseräquivalenzdurchmesser, nicht aber vom Referenzdosisparameter abhängt. Die Funktion kann insbesondere einem Wasseräquivalenzparameter einen Korrekturfaktor zuweisen. Die Funktion kann dabei insbesondere als Tabelle gegeben sein, wobei die Tabelle einer Mehrzahl von Wasseräquivalenzdurchmessern jeweils einen Korrekturfaktor zuweist, und wobei Funktionswerte für nicht tabellierte Wasseräquivalenzdurchmesser durch Interpolation, insbesondere durch lineare Interpolation bestimmt werden. Der Erfinder hat erkannt, dass sich die größenspezifische Dosisschätzung derart besonders gut und genau auf Basis des Referenzdosisparameters berechnen lässt.

Nach einem weiteren Aspekt der Erfindung wird ein Aufnahmeparameters eines Computertomographiegeräts basierend auf dem Referenzdosisparameter mittels der Recheneinheit bestimmt, wobei eine zweite Röntgendosis dem Referenzdosisparameter entspricht, wobei die zweite Röntgendosis bei Aufnahme eines zweiten CT-Bilddatensatzes in der Schnittebene absorbiert werden würde, wenn der zweite CT-Bilddatensatz unter Verwendung des Aufnahmeparameters mittels des Computertomographiegeräts aufgenommen wird. Mit anderen Worten ist es hierbei nicht notwendig, den zweiten CT-Bilddatensatz tatsächlich aufzunehmen, die zweite Röntgendosis wird nur theoretisch in der Schnittebene absorbiert, wenn unter Verwendung des Aufnahmeparameters ein zweiter CT-Bilddatensatz mittels des Computertomographiegeräts aufgenommen werden würde. Insbesondere kann der Aufnahmeparameter weiterhin auch auf dem Wasseräquivalenzdurchmesser basieren. Optional kann bei diesem Aspekt der Erfindung auch anschließend an das Bestimmen der zweite CT-Bilddatensatz mittels des Computertomographiegerätes aufgenommen werden. Der Erfinder hat erkannt, dass sich durch diesen Verfahrensschritt ein Aufnahmeparameter besonders effizient und einfach bestimmen lässt, so dass die zweite Röntgendosis möglichst nah am Referenzdosisparameter liegt, und somit der Patient einer möglichst geringen Röntgendosis ausgesetzt ist. Insbesondere kann so der Aufnahmeparameter vor der eigentlichen Untersuchung bestimmt werden. Bei einem Aufnahmeparameter kann es sich beispielsweise um die Röntgenspannung der Röntgenröhre handeln, oder um den Röntgenstrom der Röntgenröhre, oder um die Schichtdicke des zweiten CT-Bilddatensatzes, oder um den Pitchfaktor einer Spiralaufnahme, oder um weitere bekannte Parameter einer Computertomographiebildgebung.

Nach einem weiteren möglichen Aspekt der Erfindung wird weiterhin ein Planungsuntersuchungvolumen mittels der Schnittstelle empfangen. Das Planungsuntersuchungvolumen kann insbesondere das beim ersten CT-Bilddatensatz aufzunehmende Untersuchungsvolumen beschreiben. Weiterhin wird basierend auf dem Referenzdosisparameter ein Planungsdosisparameter mittels der Recheneinheit berechnet, wobei eine im Planungsuntersuchungsvolumen absorbierte Röntgendosis dem Planungsdosisparameter entspricht, wenn ein CT-Bilddatensatz des Planungsuntersuchungvolumen aufgenommen wird, wobei das Rauschen des CT-Bilddatensatzes des Planungsuntersuchungvolumen dem Rauschpegel entspricht. Weiterhin kann der Planungsdosisparameter angezeigt werden. Beim Planungsdosisparameter kann es sich insbesondere um ein Dosislängeprodukt (ein englischer Fachbegriff ist "Dose Length Product") handeln. Der Erfinder hat erkannt, dass basierend auf dem Planungsdosisparameter eine besonders effektive und genaue Planung einer Computertomographieuntersuchung erfolgen kann.

Nach einem weiteren Aspekt der Erfindung wird weiterhin ein Realdosisparameter einer Aufnahme eines dritten CT-Bilddatensatzes des Untersuchungsvolumens mittels der Schnittstelle empfangen. Weiterhin wird basierend auf einem Vergleich des Referenzdosisparameters und des Realdosisparameters die Potenz und/oder die Proportionalitätskonstante mittels der Recheneinheit korrigiert. Der Erfinder hat erkannt, dass durch die Korrektur basierend auf einem derartigen Vergleich die Proportionalitätskonstante besonders schnell und einfach korrigiert werden kann. Dies kann insbesondere dazu genutzt werden, die Proportionalitätskonstante für verschiedene Computertomographieprodukte, insbesondere für verschiedene Computertomographieprodukte unterschiedlicher Hersteller, zu bestimmen oder zu optimieren.

Die Erfindung betrifft weiterhin eine Parameterbestimmungseinheit, umfassend folgende Einheiten:

- Schnittstelle, ausgebildet zum ersten Empfangen eines Wasseräquivalenzdurchmessers einer Schnittebene eines Untersuchungsvolumens,
- Recheneinheit, ausgebildet zum ersten Bestimmen eines Rauschpegels basierend auf dem Wasseräquivalenzdurchmesser, wobei der Rauschpegel ein oberer Schwellenwert für das Rauschen eines CT-Bilddatensatzes ist,

weiterhin ausgebildet zum zweiten Bestimmen eines Referenzdosisparameters basierend auf dem Rauschpegel und dem Wasseräquivalenzdurchmesser, wobei der Referenzdosisparameter einer ersten Röntgendosis entspricht, welche bei einer Aufnahme eines ersten CT-Bilddatensatzes des Untersuchungsvolumens in der Schnittebene absorbiert werden würde, wenn das Rauschen des ersten CT-Bilddatensatzes dem Rauschpegel entspricht, wobei der Referenzdosisparameter indirekt proportional zu einer Potenz des Rauschpegels ist.

Eine solche Parameterbestimmungseinheit kann insbesondere dazu ausgebildet sein die zuvor beschriebenen erfindungsgemäßen Verfahren und ihre Aspekte auszuführen. Die Parameterbestimmungseinheit ist dazu ausgebildet diese Verfahren und ihre Aspekte auszuführen, indem die Schnittstelle und die Recheneinheit ausgebildet sind die entsprechenden Verfahrensschritte auszuführen.

Die Erfindung betrifft weiterhin ein Computertomographiegerät umfassend die Parameterbestimmungseinheit. Ein Computertomographiegerät ist insbesondere dazu ausgebildet, mittels Röntgenstrahlung ein tomographisches Schnittbild oder ein dreidimensionales Bild eines Untersuchungsvolumens zu erzeugen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Parameterbestimmungseinheit auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Ein Wasseräquivalenzdurchmesser eines Patienten beschreibt die Gesamtschwächung von Röntgenstrahlung durch den Patienten normiert auf die Schwächung von Wasser. Ein Wasseräquivalenzdurchmesser eines Patienten kann insbesondere als der Durchmesser eines Wasserzylinders definiert sein, wobei der Wasserzylinder bei einer Computertomographiebildgebung die gleiche Gesamtschwächung der Röntgenintensität verursacht wie der Patient, wenn die Schnittebene der axialen Schnittbilder orthogonal zur Symmetrieachse des Wasserzylinders angeordnet ist. Der Wasseräquivalenzdurchmesser kann auch richtungsabhängig sein, insbesondere da der Querschnitt eines Patienten im Regelfall nicht kreisförmig ist. Eine Methode zur Berechnung des Wasseräquivalenzdurchmessers auf Basis eines axialen Schnittbilde ist beispielsweise aus der Veröffentlichung American Association of Physicists in Medicine (2014): "Use of Water Equivalent Diameter for Calculating Patient Size and Size-Specific Dose Estimates (SSDE) in CT (Task Group 220)" bekannt. Weiterhin kann der Wasseräquivalenzdurchmesser auch auf Basis eines Topogramms berechnet werden, beispielweise durch eine Schwellenwertsegmentierung entlang einer Geraden.

Ein Referenzdosisparameter und/oder ein Realdosisparameter können insbesondere einem Computertomographiedosisindex (ein englischer Fachbegriff ist "computed tomography dose index", kurz "CTDI") entsprechen, insbesondere einem gewichteten Computertomographieindex (kurz "CTDI$_w$") insbesondere dem volumenbezogenen Computertomographiedosisindex (kurz "CTDI$_{vol}$") einer Spiralcomputertomographie.

Der Computertomographie-Dosisindex (kurz CTDI) kann definiert werden über die Formel

$$CTDI = \frac{1}{nT} \int_{-7T}^{7T} D(z)dz,$$

wobei T die Schichtdicke der Computertomographieaufnahme, n die Anzahl der Schichten und D(z) die am Ort z mittels eines Dosimeters in einem Phantom gemessene Dosis ist. Hierbei wird üblicherweise ein Phantom mit Durchmesser 16 cm oder mit Durchmesser 32 cm verwendet. Üblicherweise wird die Messung mit einem 100mm langen Stiftkammerdosimeter durchgeführt, und die Definition des CTDI modifiziert als

$$CTDI_{100} = \frac{1}{nT} \int_{-50mm}^{50mm} D(z)dz.$$

Da die Dosis im Patienten nahe der Oberfläche höher ist als im Inneren des Patienten, wird der gewichtete CTDI$_w$ verwendet

$$CTDI_w = \frac{1}{3}CTDI_{100}^{\mathtt{zentral}} + \frac{2}{3}CTDI_{100}^{\mathtt{peripher}},$$

wobei $CTDI_{100}^{\mathtt{zentral}}$ in der Mitte des Phantoms gemessen wird, und $CTDI_{100}^{\mathtt{peripher}}$ am Rand des Phantoms gemessen wird.

Bei vielen Computertomographiegeräten ist es möglich, einen Spiralscan durchzuführen. Hierbei wird das Untersuchungsvolumen mit einer konstanten Geschwindigkeit durch das Computertomographiegerät bewegt, während sich die Röntgenquelle mit einer konstanten Winkelgeschwindigkeit innerhalb des Computertomographiegerätes rotiert. Relativ zum Untersuchungsvolumen bewegt sich die Röntgenquelle daher auf einer Spirale, insbesondere auf einer Helix. Ein Spiralscan wird durch den Pitchfaktor p beschrieben, der als P = d / (M·T) definiert ist, wobei d die während eines vollständigen Umlaufs der Röntgenquelle durch das Untersuchungsvolumen zurückgelegte Strecke bezeichnet, und wobei M die Zahl der verwendeten Zeilen des Röntgendetektors ist, und wobei T die Schichtdicke ist. Der Pitchfaktor kann insbesondere Werte zwischen 0 und 2 annehmen. Als Maß für die Dosis bei einem Spiralscan wird der $CTDI_{vol}$ verwendet, welcher der um den Pitchfaktor korrigierte $CTDI_w$ nach der folgenden Formel ist

$$CTDI_{vol} = \frac{CTDI_w}{P}.$$

Ein Referenzdosisparameter und/oder ein Realdosisparameter können auch dem Dosislängenprodukt (ein englischer Fachbegriff ist "dose length product", kurz "DLP") entsprechen. Hierbei wird das Dosislängenprodukt insbesondere als Produkt eines Computertomographie-Dosisindex mit der axialen Länge des Untersuchungsvolumens definiert.

Neben dem Computertomographie-Dosisindex und dem Dosislängenprodukt ist auch die größenspezifische Dosisschätzung (ein englischer Fachbegriff ist "size-specific dose estimation", kurz "SSDE") bekannt. Dieser basiert auf dem Computertomographie-Dosisindex einer Computertomograhpiebildgebung und bezieht zusätzlich einen Wasseräquivalenzdurchmesser eines Patienten ein. Zur Definition der verschiedenen Dosisparameter wird insbesondere auch auf die Veröffentlichung American Association of Physicists in Medicine (2008): "The Measurement, Reporting, and Management of Radiation Dose in CT (Task Group 23)" verwiesen.

Ein Topogramm ist insbesondere eine zweidimensionale Übersichtsaufnahme des Untersuchungsvolumens mittels Röntgenstrahlung, es kann insbesondere einer zweidimensionalen Röntgenaufnahme entsprechen, insbesondere einer Durchleuchtungsaufnahme. Zur Aufnahme eines Topogramms kann ein Untersuchungsvolumen mit Hilfe des verfahrbaren Tisches am Fächerstrahl der stillstehenden Röhre vorbeigefahren werden. Bei einem Topogramm kann es sich insbesondere um eine Röntgenprojektion bezüglich einer Projektionsrichtung handeln. Ein Topogramm kann insbesondere ein laterales Topogramm oder ein anterior-posterior Topogramm sein.

Im Folgenden wird die Erfindung anhand der in den Figuren dargestellten Ausführungsbeispiele sowie anhand von Pseudocode näher beschrieben und erläutert.

Es zeigen

Fig. 1    ein Flussdiagramm eines ersten Ausführungsbeispiels des Verfahrens zum Bestimmen eines Referenzdosisparameters,

Fig. 2    ein Flussdiagramm eines zweiten Ausführungsbeispiels des Verfahrens zum Bestimmen eines Referenzdosisparameters,

Fig. 3    ein Flussdiagramm eines dritten Ausführungsbeispiels des Verfahrens zum Bestimmen eines Referenzdosisparameters,

Fig. 4    eine Parameterbestimmungseinheit,

Fig. 5    eine Parameterbestimmungseinheit in einem Umfeld für verteiltes Rechnen.

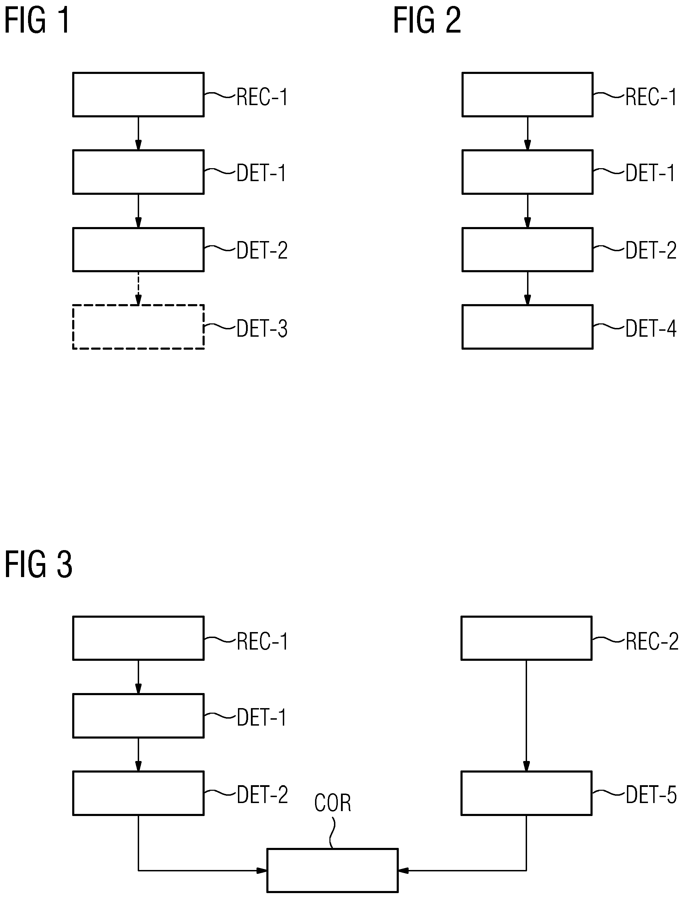

Fig. 1 zeigt ein Flussdiagramm eines ersten Ausführungsbeispiels des Verfahrens zum Bestimmen eines Referenzdosisparameters.

Der erste Schritt des dargestellten ersten Ausführungsbeispiels ist das erstes Empfangen REC-1 eines Wasseräquivalenzdurchmessers einer Schnittebene eines Untersuchungsvolumens mittels einer Schnittstelle 401. Der Wasseräquivalenzdurchmesser kann insbesondere vorher basierend auf einem Topogramm des Untersuchungsvolumens aufgenommen worden sein.

Der zweite Schritt des dargestellten ersten Ausführungsbeispiels ist das erste Bestimmen DET-1 eines Rauschpegels basierend auf dem Wasseräquivalenzdurchmesser mittels einer Recheneinheit 402, wobei der Rauschpegel ein oberer Schwellenwert für das Rauschen eines CT-Bilddatensatzes ist. Das Rauschen eines CT-Bildes wird in diesem Ausführungsbeispiel als Standardabweichung der gemessenen Hounsfieldeinheiten eines Bereiches, der konstante reale Hounsfieldeinheiten aufweist, definiert. Bei diesem Bereich mit konstanten realen Hounsfieldeinheiten kann es sich um die im Bilddatensatz dargestellte Luft handeln, alternativ kann das Rauschen auch immer durch Computertomographiebildgebung eines zumindest teilweise homogenen Phantoms bestimmt werden. Im dargestellten Ausführungsbeispiel wird der Rauschpegel s durch die Formel

$$s = \max(c, \ m \cdot WED + t)$$

bestimmt, wobei hier die Konstanten als c = 4 HU, m = 1 HU/cm und t = - 14 HU gewählt werden. Es ist natürlich auch möglich, abweichende Konstanten zu wählen, um veränderten Anforderungen an die Berechnung gerecht zu werden. Weiterhin bezeichnet hier max(a,b) das Maximum von einer Größe a und einer Größe b, in anderen Worten ist max(a,b) = a, wenn a größer oder gleich b ist, und max(a,b) = b, wenn b größer als a ist.

Der nächste Schritt des dargestellten ersten Ausführungsbeispiels ist das zweite Bestimmen DET-2 eines Referenzdosisparameters basierend auf dem Rauschpegel und dem Wasseräquivalenzdurchmesser mittels der Recheneinheit 402, wobei der Referenzdosisparameter einer ersten Röntgendosis entspricht, welche bei einer Aufnahme eines ersten CT-Bilddatensatzes des Untersuchungsvolumens in der Schnittebene absorbiert werden würde, wenn das Rauschen des ersten CT-Bilddatensatzes dem Rauschpegel entspricht, wobei der Referenzdosisparameter indirekt proportional zu einer Potenz des Rauschpegels ist. Der Referenzdosisparameter ist in diesem Ausführungsbeispiel ein Computertomograhpiedosisindex (ein englischer Fachbegriff ist "computed tomography dose index", kurz "CTDI"), insbesondere der $CTDI_{vol}$ eines Spiralscans.

Im dargestellten Ausführungsbeispiel wird der Referenzdosisparameter berechnet als

$$CTDI_{vol}(WED, s) = k \cdot \frac{\exp(l_w \cdot WED)}{s},$$

wobei hier der Wert p der Potenz genau 1 ist, wobei k eine Proportionalitätskonstante ist, die in diesem Ausführungsbeispiel als k = 1 mGy·HU gewählt wird, und $l_w$ der linearen Abschwächungskoeffizienten von Röntgenstrahlung in Wasser ist. Hierbei wird üblicherweise $l_w$ = 0,19 cm$^{-1}$ als linearen Abschwächungskoeffizient angenommen. Alternativ kann der Referenzdosisparameter auch berechnet werden als

$$CTDI_{vol}(WED, s) = k \cdot \exp(l_w \cdot WED) \cdot \left(\frac{s_0}{s}\right)^p,$$

wobei $s_0$ ein Skalierungsfaktor des Rauschpegels ist, wobei die Einheit des Skalierungsfaktors des Rauschpegels ebenfalls HU bzw. Hounsfeldeinheit ist. In diesem Fall trägt die Proportionalitätskonstante k die Einheit mGy. Wenn bei dieser alternativen Berechnungsmethode $s_0$ = 1 HU und p = 1 gewählt wird, sind beide Berechnungsmethoden identisch.

Der nächste Schritt des dargestellten Ausführungsbeispiels ist das dritte Bestimmen DET-3 einer größenspezifischen Dosisschätzung basierend auf dem Referenzdosisparameter mittels der Recheneinheit 402. Hierbei handelt es sich um einen optionalen Schritt, der nicht in jedem Ausführungsbeispiel des erfindungsgemäßen Verfahrens implementiert sein muss.

Die größenspezifische Dosisschätzung wird in diesem Ausführungsbeispiel berechnet als

$$SSDE(WED, s) = f(WED) \cdot CTDI_{vol}(WED, s),$$

wobei f eine Funktion ist, die nur vom Wasseräquivalenzdurchmesser WED abhängt, nicht aber vom $CTDI_{vol}$. Die Funktion f ist in diesem Ausführungsbeispiel durch eine Tabelle gegeben, welche die Funktionswerte für mehrere Werte des Wasseräquivalenzdurchmessers umfasst. Diese Funktionswerte werden oftmals auch als Korrekturfaktoren bezeichnet. Ein Funktionswert für einen nicht-tabellierten Wasseräquivalenzdurchmesser kann durch Interpolation, insbesondere durch lineare Interpolation von zwei oder mehreren Funktionswerten von tabellierten Wasseräquivalenzdurchmessern bestimmt werden.

Dieses Ausführungsbeispiels kann verwendet werden, um bei einer Computertomograpiebildgebung den Realdosisparameter, hier insbesondere der durch die konkrete Bildgebung gegebene $CTDI_{vol}$, mit dem Referenzdosisparameter, hier dem basierend auf dem Wasseräquivalenzdurchmesser berechneten $CTDI_{vol}$, zu vergleichen und so die Bildgebung im Hinblick auf die vom Patienten absorbierte Dosis zu bewerten. Diese Bewertung kann insbesondere vorgenommen werden, ohne die bei der Computertomographiebildgebung aufgenommenen axialen Bilddaten abzurufen oder zu analysieren. Daher ist dieses Ausführungsbeispiel insbesondere auch dazu geeignet, eine große Anzahl von Computertomograhpiebildgebungen zu bewerten, wobei die Daten der Computertomographiebildgebungen räumlich getrennt von der Auswerteeinheit gespeichert sind. Alternativ kann die Bewertung auch jeweils bezüglich der größenspezifischen Dosisschätzung durchgeführt werden.

| Tabelle A: Pseudocode zum ersten Ausführungsbeispiel | |
|---|---|
| A.1 | function noise(WED): |
| A.2 | m = 1.0; t = -14.0; c = 4.0 |
| A.3 | return max(c, m*WED + t) |
| | |
| A.4 | function ctdi_vol (WED): |
| A.5 | k = 1.0; l_water = 0.19 |
| A.6 | return k*exp(l_water * WED) / noise(WED) |
| | |
| A.7 | function ssde(WED, cor_factors): |
| A.8 | WED_1 = lower_key(cor_factors, WED) |
| A.9 | WED_2 = upper_key(cor_factors, WED) |
| A.10 | cor_ factor = (cor_ factors[WED_ 2] - cor_ factors[WED_ 1]) * (WED - WED_1)/(WED_ 2 - WED_1) + cor_factors[WED_1] |
| | |
| A.11 | return cor factor*WED |

Tabelle A zeigt Pseudocode für das erste Ausführungsbeispiel. In den Codezeilen A.1, A.4 und A.7 sind die Funktionsdeklarationen zur Bestimmung des Rauschpegels, des $CTDI_{vol}$ und des SSDE aufgeführt. In den Codezeilen A.2 und A.5 werden die konstanten Parameter wie zum ersten Ausführungsbeispiel beschrieben gewählt. Die Codezeilen A.3 und A.6 entsprechen den bereits beschriebenen Formeln zur Bestimmung des Rauschpegels und des $CTDI_{vol}$. Der Funktion "ssde" wird neben dem Wasseräquivalenzdurchmesser auch eine Liste "cor_factors" mit Paaren von Wasseräquivalenzdurchmessern und zugehörigen Korrekturfaktoren übergeben. Durch die Funktion "lower_key" in der Codezeile A.8 kann der größte Wasseräquivalenzdurchmesser der Liste "cor_factors" bestimmt werden, der noch kleiner als der an die Funktion übergebene Wasseräquivalenzdurchmesser ist. Durch die Funktion "upper_key" in der Codezeile A.9 kann der kleinste Wasseräquivalenzdurchmesser der Liste "cor_factors" bestimmt werden, der noch größer als der an die Funktion übergebene Wasseräquivalenzdurchmesser ist. An dieser Stelle kann optional noch eine Abfrage eingebaut werden, ob der an die Funktion übergebene Wasseräquivalenzdurchmesser in der Liste "cor_factors" enthalten ist, und der zugeordnete Korrekturfaktor mit dem an die Funktion übergebenen Wasseräquivalenzdurchmesser multipliziert zurückgegeben werden. Falls die Abfrage negativ ist, oder in dem aufgeführten Pseudocode der Tabelle A immer, wird in der Codezeile A.10 eine lineare Interpolation des Korrekturfaktors "cor_factor" berechnet, der in Codezeile A.11 mit dem an die Funktion übergebenen Wasseräquivalenzdurchmesser multipliziert wird.

Fig. 2 zeigt ein Flussdiagramm eines zweiten Ausführungsbeispiels des Verfahrens zum Bestimmen eines Referenzdosisparameters. Die Verfahrensschritte des ersten Empfangens REC-1, des ersten Bestimmens DET-2 und des zweiten Bestimmens DET-3 werden analog zu der Beschreibung des ersten Ausführungsbeispiels durchgeführt.

Ein weiterer Schritt des dargestellten zweiten Ausführungsbeispiels ist das vierte Bestimmen DET-4 eines Aufnahmeparameters eines Computertomographiegeräts 420 basierend auf dem Referenzdosisparameter mittels der Recheneinheit 402, wobei eine zweite Röntgendosis dem Referenzdosisparameter entspricht, wobei die zweite Röntgendosis bei Aufnahme eines zweiten CT-Bilddatensatzes in der Schnittebene absorbiert werden würde, wenn der zweite CT-Bilddatensatz unter Verwendung des Aufnahmeparameters mittels des Computertomographiegeräts 420 aufgenommen wird. Im dargestellten Ausführungsbeispiel ist der Aufnahmeparameter der Röntgenstrom der Röntgenröhre des Computertomographiegerätes 420. Umfasst das Computertomographiegerät 420 Mittel zur Dosismodulation, insbesondere durch Variation des Röntgenstroms, so kann der Aufnahmeparameter alternativ auch ein Mittelwert des Röntgenstroms der Röntgenröhre des Computertomographiegerätes 420 sein.

Ist der Röntgenstrom sowie alle weiteren die Computertomographie beeinflussenden Größen (wie beispielsweise Pitchfaktor oder Röntgenspannung) vorgegeben, ist es möglich, den $CTDI_{vol}$ einer Bildgebung unter Verwendung des Röntgenstroms und der weiteren Größen zu bestimmen. Beispielsweise kann der $CTDI_{vol}$ auf Grundlage von tabellierten Messwerten berechnet werden, wobei die Messwerte mittels einem oder mehreren Dosimetern in einem Phantom aufgezeichnet wurden. Sind zu einer spezifischen Kombination aus Röntgenstrom und den weiteren Größen keine Messwerte tabelliert, so können diese interpoliert, insbesondere linear interpoliert werden. Insbesondere kann also, wenn die anderen Größen konstant gehalten werden, eine Funktion bestimmt werden, die den $CTDI_{vol}$ in Abhängigkeit des Röntgenstroms beschreibt.

Da die im Untersuchungsvolumen absorbierte Röntgendosis direkt proportional zum Röntgenstrom ist, steigt auch der $CTDI_{vol}$ monoton mit dem Röntgenstrom. Daher ist es möglich, den Röntgenstrom so zu bestimmen, dass der $CTDI_{vol}$ dem Referenzdosisparameter entspricht, indem die Umkehrfunktion bestimmt wird, welche den Röntgenstrom in Abhängigkeit des $CTDI_{vol}$ beschreibt. Die Bestimmung der Umkehrfunktion kann insbesondere auch graphisch oder numerisch durchgeführt werden, so dass es nicht notwendig ist, den ursprünglichen Funktionsterm zu kennen.

| Tabelle B: Pseudocode zum dritten Ausführungsbeispiel | |
|---|---|
| B.1 | function xray_current_it(ctdi_vol, imaging_params, current_lower, current_upper): |
| B.2 | ctdi_vol_calc_lower = scanner.ctdi_vol(imaging_params, current_lower) |
| B.3 | ctdi_vol_calc_upper = scanner.ctdi_vol(imaging_params, current_upper) |
| B.4 | current_mid = (current_lower + current_upper)/2 |
| B.5 | ctdi_vol_calc_mid = scanner.ctdi_vol(xray_current, current_mid) |
| B.6 | if abs(ctdi_vol_calc_mid - ctdi_vol)/ ctdi_vol < 0.01: |
| B.7 | return current_mid |
| B.8 | else if ctdi_vol_calc_mid > ctdi vol: |
| B.9 | return xray_current_it(ctdi_vol, imaging_params, current_lower, current_mid) |
| B.10 | else: |
| B.11 | return xray_current_it(ctdi_vol, imaging_params, current_mid, current_upper) |
| B.12 | function xray_current(WED, imaging_params): |
| B.13 | ctdi_vol_ref = ctdi_vol(WED) |
| B.14 | current_max = scanner.xray_current_max |
| B.15 | return xray_current_it(ctdi_vol_ref, imaging_params, 0, current_max) |

Tabelle B zeigt Pseudocode für das zweite Ausführungsbeispiel. In den Codezeilen B.1, ..., B.11 ist eine Funktion zum iterativen Bestimmen des Röntgenstroms definiert, die auf dem Bisektionsverfahren basiert. In den Codezeilen B.12, ..., B.15 ist eine Funktion zum Bestimmen des Röntgenstroms basierend auf dem Wasseräquivalenzdurchmesser "WED" und den weiteren Größen "imaging_params" definiert.

In Codezeile B.13 wird der Referenz-$CTDI_{vol}$ mittels der in der Tabelle A definierten Funktion berechnet. In der Codezeile B.14 wird der maximale Röntgenstrom "current_max" des Scanners abgerufen. Codezeile B.15 ruft die iterative Funktion zum Bestimmen des Röntgenstroms auf.

In den Codezeilen B.2 und B.3 wird der $CTDI_{vol}$ mittels einer vom Scanner zur Verfügung gestellten Funktion berechnet, wobei zwei unterschiedliche Röntgenströme "current_lower" und "current_upper" verwendet werden, so dass der $CTDI_{vol}$ im ersten Fall kleiner als der Referenz-$CTDI_{vol}$ und im zweiten Fall größer als der Referenz-$CTDI_{vol}$ ist. Die vom Scanner zur Verfügung gestellte Funktion zur Berechnung des $CTDI_{vol}$ berechnet diesen Dosisparameter in Abhängigkeit der gegebenen Aufnahmeparameter.

In der Codezeile B.4 wird ein weiterer Röntgenstrom "current_mid" als Mittelwert der Röntgenströme "current_lower" und "current_upper" bestimmt, weiterhin wird in Codezeile B.5 der $CTDI_{vol}$ bei diesem Röntgenstrom bestimmt.

Falls der $CTDI_{vol}$ beim Mittelwert der Röntgenströme "current_lower" und "current_upper" nahe genug am Referenz-$CTDI_{vol}$ ist (in dem hier gezeigten Pseudocode, wenn die relative Abweichung kleiner als 1 Prozent ist), wird in Codezeile B.6 und B.7 dieser Mittelwert des Röntgenstroms zurückgegeben. Andernfalls wird in den Codezeilen B.8, ..., B.11 die Funktion iterativ aufgerufen, wobei die Funktionsparameter der Röntgenströme basierend auf dem $CTDI_{vol}$ beim Mittelwert des Röntgenstroms bestimmt werden.

Fig. 3 zeigt ein Flussdiagramm eines dritten Ausführungsbeispiels des Verfahrens zum Bestimmen eines Referenzdosisparameters. Die Verfahrensschritte des ersten Empfangens REC-1, des ersten Bestimmens DET-2 und des zweiten Bestimmens DET-3 werden analog zu der Beschreibung des ersten Ausführungsbeispiels durchgeführt.

Weiterhin erfolgt im dritten Ausführungsbeispiel ein zweites Empfangen REC-2 eines Realdosisparameters einer Aufnahme eines dritten CT-Bilddatensatzes des Untersuchungsvolumens mittels der Schnittstelle 401, wobei der Realdosisparameter der bei der Aufnahme des dritten CT-Bilddatensatzes in der Schnittebene absorbierten dritten Röntgendosis entspricht. Weiterhin wird beim zweiten Empfangen REC-2 auch der Rauschpegel des dritten CT-Bilddatensatzes empfangen.

Weiterhin erfolgt im dritten Ausführungsbeispiel ein Korrigieren COR der Potenz und/oder der Proportionalitätskonstante basierend auf einem Vergleich des Referenzdosisparameters und des Realdosisparameters mittels der Recheneinheit 402. Ein Korrigieren kann insbesondere durch Anpassung einer Funktionsgleichung realisiert werden, bei der die Potenz und/oder die Proportionalitätskonstante als Fitparameter aufgefasst werden. Das Anpassen einer Funktionsgleichung kann insbesondere mittels des Levenberg-Marquardt-Algorithmus durchgeführt werden. Die Funktionsgleichung kann insbesondere durch die in den Ausführungen zum ersten Ausführungsbeispiel definierte Funktion $CTDI_{vol}(WED,s)$ gegeben sein. Bei der Anpassung der Funktionsgleichung können insbesondere auch weitere Paare von Referenzdosisparametern und Realdosisparametern herangezogen werden.

Das dritte dargestellte Ausführungsbeispiel ist insbesondere dann vorteilhaft, wenn die Potenz und/oder die Proportionalitätskonstante für verschiedene Computertomograhpiegeräte oder unterschiedliche Hersteller jeweils separat gewählt werden müssen.

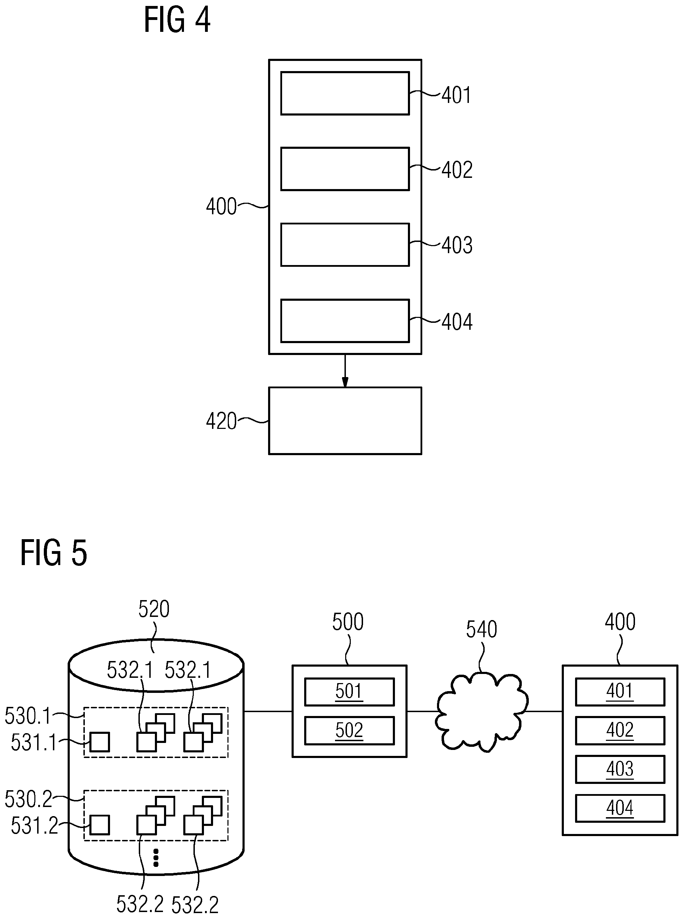

Fig. 4 zeigt eine Parameterbestimmungseinheit 400 zur Bestimmung eines Referenzdosisparameters. Die hier gezeigte Parameterbestimmungseinheit 400 ist ausgelegt, ein erfindungsgemäßes Verfahren auszuführen. Diese Parameterbestimmungseinheit 400 umfasst eine Schnittstelle 401, eine Recheneinheit 402, eine Speichereinheit 403 sowie eine Ein- und Ausgabeeinheit 404.

Bei der Parameterbestimmungseinheit 400 kann es sich insbesondere um einen Computer, einen Mikrocontroller oder um einen integrierten Schaltkreis handeln. Alternativ kann es sich bei der Parameterbestimmungseinheit 400 um einen realen oder virtuellen Verbund von Computern handeln (ein englischer Fachbegriff für einen realen Verbund ist "Cluster", ein englischer Fachbegriff für einen virtuellen Verbund ist "Cloud").

Bei einer Schnittstelle 401 kann es sich um eine Hardware- oder Softwareschnittstelle handeln (beispielsweise PCI-Bus, USB oder Firewire). Eine Recheneinheit 402 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit 403 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Eine Ein- und Ausgabeeinheit 404 umfasst wenigstens eine Eingabeeinheit und/oder wenigstens eine Ausgabeeinheit.

Fig. 5 zeigt ein Ausführungsbeispiel einer möglichen Einbindung einer Parameterbestimmungseinheit 400 in eine Umgebung für verteiltes Rechnen. Die Parameterbestimmungseinheit ist über ein Netzwerk 540 mit einem Server 500 verbunden, der wiederum mit einer Datenbank 520 verbunden ist.

Bei einem Server 500 kann es sich insbesondere um einen Computer, einen Mikrocontroller oder um einen integrierten Schaltkreis handeln. Alternativ kann es sich bei dem Server 500 um einen realen oder virtuellen Verbund von Computern handeln (ein englischer Fachbegriff für einen realen Verbund ist "Cluster", ein englischer Fachbegriff für einen virtuellen Verbund ist "Cloud").

Der Server umfasst eine Serverschnittstelle 501 und eine Serverrecheneinheit 502. Bei einer Serverschnittstelle 501 kann es sich um eine Hardware- oder Softwareschnittstelle handeln (beispielsweise PCI-Bus, USB oder Firewire). Eine Serverrecheneinheit 502 kann Hardware-Elemente oder Software-Elemente aufweisen, beispielsweise einen Mikroprozessor oder ein sogenanntes FPGA (englisches Akronym für "Field Programmable Gate Array").

Die Datenbank 520 kann in einem Speicher des Servers 500 realisiert sein, sie kann aber auch wie im dargestellten Ausführungsbeispiel als separater Datenbankserver ausgebildet sein. Die Datenbank 520 umfasst eine Mehrzahl von Untersuchungsdatensätzen 530.1, 530.2, jeder der Untersuchungsdatensätze 530.1, 520.2 umfasst eine Topogrammaufnahme 531.1, 531.2 eines Untersuchungsbereichs sowie eine oder mehrere Bilderserien 532.1, 532.2 umfassend axiale Schnittbilder des Untersuchungsbereichs.

Bei einem Netzwerk 540 kann es sich um ein lokales Netzwerk (ein englischer Fachbegriff ist "local area network", kurz "LAN") oder um ein Weitverkehrsnetz (ein englischer Fachbegriff ist "wide area network", kurz "WAN") handeln. Ein Beispiel für ein LAN ist ein Intranet, ein Beispiel für ein WAN ist das Internet. Die Verbindung kann dabei kabelgebunden (beispielsweise über Ethernet, PowerLAN oder TokenRing) erfolgen, alternativ kann die Verbindung auch kabellos erfolgen (beispielsweise über "wireless local area network", kurz "WLAN", oder Infrarot oder Bluetooth). Im dargestellten Ausführungsbeispiel entspricht das Netzwerk 540 dem Internet.

Im dargestellten Ausführungsbeispiel sind der Server 500 und die Datenbank 520 Teil einer Architektur für verteiltes Rechnen. Die Datenbank 520 speichert Daten von einer großen Anzahl von bildgebenden Computertomographiebildgebungen, der Server 500 regelt den Zugriff auf die Datenbank 520. Wie im ersten Ausführungsbeispiel soll von der Vielzahl der Computertomographiebildgebungen der Referenzwert des $CTDI_{vol}$ und/oder der SSDE berechnet werden.

Aus dem Stand der Technik ist bekannt, die Bildserien 532.1, 532.2 der Untersuchungsdatensätze 530.1, 530.2 über das Netzwerk 540 an die Parameterbestimmungseinheit zu übertragen, um aus den Bildserien 532.1, 532.2 umfassend axiale Schnittbilder den jeweiligen $CTDI_{vol}$ und/oder den SSDE zu berechnen. Die Berechnung auf Basis der axialen Schnittbilder ist aber langsam, ebenso müssen über die beschränkte Datenbandbreite des Netzwerks 540 hohe Datenmengen übertragen werden.

Bei dem dargestellten Ausführungsbeispiel der vorliegenden Erfindung wird nur wenigstens eine Topgrammaufnahme 531.1, 531.2 über das Netzwerk 540 an die Parameterbestimmungseinheit 400 übertragen, jedoch keine Bildserien 532.1, 532.2 umfassend axiale Schnittbilder. Aus einer Topogrammaufnahme 531.1, 531.2 kann ein Wasseräquivalenzparameter einer Schnittebene bestimmt werden, indem die Topogrammaufnahme 531.1, 531.2 mit Referenztopogrammaufnahmen verglichen wird, zu denen jeweils der Wasseräquivalenzdurchmesser bekannt ist, oder mittels einer Schwellenwertsegmentierung. Unter Verwendung des im Fig. 1 dargestellten Flussdiagramms kann dann der optimale $CTDI_{vol}$ der jeweiligen Aufnahme berechnet werden, und dieser Referenzdosisparameter für Statistiken über viele Computertomographiebildgebungen verwendet werden.

## Patentansprüche

1. Verfahren zum Bestimmen eines Referenzdosisparameters einer Computertomographiebildgebung, umfassend folgende Verfahrensschritte:

   - Erstes Empfangen (REC-1) eines Wasseräquivalenzdurchmessers einer Schnittebene eines Untersuchungsvolumens mittels einer Schnittstelle (401),
   - Erstes Bestimmen (DET-1) eines Rauschpegels basierend auf dem Wasseräquivalenzdurchmesser mittels einer Recheneinheit (402),
   wobei der Rauschpegel ein oberer Schwellenwert für das Rauschen eines CT-Bilddatensatzes ist,
   - Zweites Bestimmen (DET-2) eines Referenzdosisparameters basierend auf dem Rauschpegel und dem Wasseräquivalenzdurchmesser mittels der Recheneinheit (402),
   wobei der Referenzdosisparameter einer ersten Röntgendosis entspricht, welche bei einer Aufnahme eines ersten CT-Bilddatensatzes des Untersuchungsvolumens in der Schnittebene absorbiert werden würde, wenn das Rauschen des ersten CT-Bilddatensatzes dem Rauschpegel entspricht,
   wobei der Referenzdosisparameter indirekt proportional zu einer Potenz des Rauschpegels ist.

2. Verfahren nach Anspruch 1, wobei die Potenz einen Wert zwischen 0.1 und 2.5, insbesondere zwischen 0.5 und 1.5, insbesondere zwischen 0.9 und 1.1 aufweist.

3. Verfahren nach einem der vorherigen Ansprüche, wobei der Referenzdosisparameter weiterhin proportional zu einer Exponentialfunktion des Wasseräquivalenzdurchmessers ist.

4. Verfahren nach Anspruch 3, wobei das Argument der Exponentialfunktion das Produkt des linearen Abschwächungskoeffizienten von Röntgenstrahlung in Wasser mit dem Wasseräquivalenzdurchmesser umfasst.

5. Verfahren nach Anspruch 3 oder 4, wobei der Referenzdosisparameter das Produkt einer Proportionalitätskonstante mit dem Quotienten der Exponentialfunktion und der Potenz des Rauschpegels ist, wobei p der Wert der Potenz ist, wobei die Proportionalitätskonstante zwischen 0,2 mGy·HU$^p$ und 2,0 mGy·HU$^p$ beträgt, oder wobei die Proportionalitätskonstante insbesondere zwischen 0,5 mGy·HU$^p$ und 1,5 mGy·HU$^p$ beträgt, oder wobei die Proportionalitätskonstante insbesondere zwischen 1,2 mGy·HU$^p$ und 1,2 mGy·HU$^p$ beträgt.

6. Verfahren nach einem der vorherigen Ansprüche, wobei der Rauschpegel eine stückweise lineare Funktion des Wasseräquivalenzdurchmessers ist.

7. Verfahren nach Anspruch 6, wobei der Rauschpegel eine stetige, abschnittsweise definierte Funktion des Wasseräquivalenzdurchmessers mit genau zwei Abschnitten ist, wobei der erste Abschnitt eine konstante Funktion ist und wobei der zweite Abschnitt eine lineare Funktion des Wasseräquivalenzdurchmessers ist.

8. Verfahren nach einem der vorherigen Ansprüche, weiterhin umfassend den folgenden Verfahrensschritt:

- Drittes Bestimmen (DET-3) einer größenspezifischen Dosisschätzung basierend auf dem Referenzdosisparameter mittels der Recheneinheit (402).

9. Verfahren nach einem der vorherigen Ansprüche, weiterhin umfassend den folgenden Verfahrensschritt:

- Viertes Bestimmen (DET-4) eines Aufnahmeparameters eines Computertomographiegeräts (420) basierend auf dem Referenzdosisparameter mittels der Recheneinheit (402),
wobei eine zweite Röntgendosis dem Referenzdosisparameter entspricht, wobei die zweite Röntgendosis bei Aufnahme eines zweiten CT-Bilddatensatzes in der Schnittebene absorbiert werden würde, wenn der zweite CT-Bilddatensatz unter Verwendung des Aufnahmeparameters mittels des Computertomographiegeräts (420) aufgenommen wird.

10. Verfahren nach einem der Ansprüche 1 bis 8, weiterhin umfassend folgende Verfahrensschritte:

- Zweites Empfangen (REC-2) eines Realdosisparameters einer Aufnahme eines dritten CT-Bilddatensatzes des Untersuchungsvolumens mittels der Schnittstelle (401),
wobei der Realdosisparameter der bei der Aufnahme des dritten CT-Bilddatensatzes in der Schnittebene absorbierten dritten Röntgendosis entspricht,
- Korrigieren (COR) der Potenz und/oder der Proportionalitätskonstante basierend auf einem Vergleich des Referenzdosisparameters und des Realdosisparameters mittels der Recheneinheit (402).

11. Parameterbestimmungseinheit (400), umfassend folgende Einheiten:

- Schnittstelle (401), ausgebildet zum ersten Empfangen (REC-1) eines Wasseräquivalenzdurchmessers einer Schnittebene eines Untersuchungsvolumens,
- Recheneinheit (402), ausgebildet zum ersten Bestimmen (DET-1) eines Rauschpegels basierend auf dem Wasseräquivalenzdurchmesser, wobei der Rauschpegel ein oberer Schwellenwert für das Rauschen eines CT-Bilddatensatzes ist,
weiterhin ausgebildet zum zweiten Bestimmen (DET-2) eines Referenzdosisparameters basierend auf dem Rauschpegel und dem Wasseräquivalenzdurchmesser, wobei der Referenzdosisparameter einer ersten Röntgendosis entspricht, welche bei einer Aufnahme eines ersten CT-Bilddatensatzes des Untersuchungsvolumens in der Schnittebene absorbiert werden würde, wenn das Rauschen des ersten CT-Bilddatensatzes dem Rauschpegel entspricht, wobei der Referenzdosisparameter indirekt proportional zu einer Potenz des Rauschpegels ist.

12. Parameterbestimmungseinheit (400), weiterhin ausgebildet ein Verfahren nach einem der Ansprüche 2 bis 10 auszuführen.

13. Computertomographiegerät (420) umfassend eine Parameterbestimmungseinheit (400) nach Anspruch 11 oder 12.

14. Computerprogrammprodukt mit einem Computerprogramm, welches direkt in einen Speicher (403) einer Parameterbestimmungseinheit (400) ladbar ist, mit Programmabschnitten, um alle Schritte des Verfahrens nach einem der Ansprüche 1 bis 10 auszuführen, wenn die Programmabschnitte von der Parameterbestimmungseinheit (400) ausgeführt werden

15. Computerlesbares Speichermedium, auf welchem von einer Parameterbestimmungseinheit (400) lesbare und ausführbare Programmabschnitte gespeichert sind, um alle Schritte des Verfahrens nach einem der Ansprüche 1 bis 10 auszuführen, wenn die Programmabschnitte von der Parameterbestimmungseinheit (400) ausgeführt werden.

**Claims**

1. Method for determining a reference dose parameter of a computed tomography imaging, comprising the following method steps:

- First receipt (REC-1) of a water equivalent diameter of a slice plane of an examination volume by means of an interface (401),
- First determination (DET-1) of a noise level based on the water equivalent diameter by means of a computer unit (402),

wherein the noise level is an upper threshold value for the noise of a CT image dataset,
- Second determination (DET-2) of a reference dose parameter based on the noise level and the water equivalent diameter by means of the computer unit (402),
wherein the reference dose parameter corresponds to a first x-ray dose, which would be absorbed in the slice plane during a recording of a first CT image dataset of the examination volume if the noise of the first CT image dataset corresponds to the noise level,
wherein the reference dose parameter is indirectly proportional to a power of the noise level.

2. Method according to claim 1, wherein the power has a value of between 0.1 and 2.5, in particular of between 0.5 and 1.5, in particular of between 0.9 and 1.1.

3. Method according to one of the preceding claims, wherein the reference dose parameter is further proportional to an exponential function of the water equivalent diameter.

4. Method according to claim 3, wherein the argument of the exponential function comprises the product of the linear attenuation coefficient of x-ray radiation in water with the water equivalent diameter.

5. Method according to claim 3 or 4, wherein the reference dose parameter is the product of a proportionality constant with the quotient of the exponential function and the power of the noise level, wherein p is the value of the power, wherein the proportionality constant amounts to between 0.2 mGy·HU$^p$ and 2.0 mGy·HU$^p$, or wherein the proportionality constant in particular amounts to between 0.5 mGy·HU$^p$ and 1.5 mGy·HU$^p$, or wherein the proportionality constant in particular amounts to between 1.2 mGy·HU$^p$ and 1.2 mGy·HU$^p$.

6. Method according to one of the preceding claims, wherein the noise level is a piecewise linear function of the water equivalent diameter.

7. Method according to claim 6, wherein the noise level is a continuous sectionally-defined function of the water equivalent diameter with precisely two sections, wherein the first section is a constant function and wherein the second section is a linear function of the water equivalent diameter.

8. Method according to one of the preceding claims, further comprising the following method step:

- Third determination (DET-3) of a size-specific dose estimation based on the reference dose parameter by means of the computer unit (402).

9. Method according to one of the preceding claims, further comprising the following method step:

- Fourth determination (DET-4) of a recording parameter of a computed tomography device (420) based on the reference dose parameter by means of the computer unit (402),
wherein a second x-ray dose corresponds to the reference dose parameter, wherein the second x-ray dose would be absorbed in the slice plane during recording of a second CT image dataset if the second CT image dataset is recorded using the recording parameter by means of the computed tomography device (420).

10. Method according to one of claims 1 to 8, further comprising the following method steps:

- Second receipt (REC-2) of a real dose parameter of a recording of a third CT image dataset of the examination volume by means of the interface (401),
wherein the real dose parameter corresponds to the third x-ray dose absorbed in the slice plane during the recording of the third CT image dataset,
- Correction (COR) of the power and/or of the proportionality constant based on a comparison of the reference dose parameter and the real dose parameter by means of the computer unit (402).

11. Parameter determination unit (400), comprising the following units:

- An interface (401), embodied for first receipt (REC-1) of a water equivalent diameter of a slice plane of an examination volume,
- A computer unit (402), embodied for first determination (DET-1) of a noise level based on the water equivalent diameter, wherein the noise level is an upper threshold value for the noise of a CT image dataset,

further embodied for second determination (DET-2) of a reference dose parameter based on the noise level and the water equivalent diameter, wherein the reference dose parameter corresponds to a first x-ray dose, which would be absorbed in the slice plane during a recording of a first CT image dataset of the examination volume if the noise of the first CT image dataset corresponds to the noise level, wherein the reference dose parameter is indirectly proportional to a power of the noise level.

12. Parameter determination unit (400), further embodied for carrying out a method according to one of claims 2 to 10.

13. Computed tomography device (420) comprising a parameter determination unit (400) according to claim 11 or 12.

14. Computer program product with a computer program, which is able to be loaded directly into a memory (403) of a parameter determination unit (400), with program sections for carrying out all steps of the method according to one of claims 1 to 10 when the program sections are executed by the parameter determination unit (400).

15. Computer-readable storage medium, on which program sections able to be read and executed by a parameter determination unit (400) are stored, in order to carry out all steps of the method according to one of claims 1 to 10 when the program sections are executed by the parameter determination unit (400).

## Revendications

1. Procédé de détermination d'un paramètre de dose de référence d'une imagerie tomographique par ordinateur, comprenant les stades de procédé suivants :

   - première réception (REC-1) d'un diamètre équivalent d'eau d'un plan de coupe d'un volume à étudier au moyen d'une interface (401),
   - première détermination (DET-1) d'un niveau de bruit reposant sur le diamètre équivalent d'eau, au moyen d'une unité (402) de calcul,
   le niveau de bruit étant une valeur de seuil supérieure du bruit d'un jeu de données d'image CT,
   - deuxième détermination (DET-2) d'un paramètre de dose de référence reposant sur le niveau de bruit et le diamètre équivalent d'eau, au moyen de l'unité (402) de calcul,
   dans lequel le paramètre de dose de référence correspond à une première dose de rayons X, qui, lors d'un enregistrement d'un premier jeu de données d'image CT du volume à étudier, aurait été absorbée dans le plan de coupe si le bruit du premier jeu de données d'image CT correspond au niveau de bruit,
   dans lequel le paramètre de dose de référence est inversement proportionnel à une puissance du niveau de bruit.

2. Procédé suivant la revendication 1, dans lequel la puissance a une valeur comprise entre 0,1 et 2,5, notamment entre 0,5 et 1,5, notamment entre 0,9 et 1,1.

3. Procédé suivant l'une des revendications précédentes, dans lequel le paramètre de dose de référence est, en outre, proportionnel à une fonction exponentielle du diamètre équivalent d'eau.

4. Procédé suivant la revendication 3, dans lequel l'argument de la fonction exponentielle comprend le produit du coefficient de l'affaiblissement linéaire du rayonnement X dans l'eau par le diamètre équivalent d'eau.

5. Procédé suivant la revendication 3 ou 4, dans lequel le paramètre de dose de référence est le produit d'une constante de proportionnalité par le quotient de la fonction exponentielle à la puissance du niveau de bruit, p étant la valeur de la puissance, la constante de proportionnalité étant comprise entre $0,2$ mGy·HU$^p$ et $2,0$ mGy·HU$^p$ ou dans lequel la constante de proportionnalité est comprise notamment entre $0,5$ mGy·HU$^p$ et $1,5$ mGy·HU$^p$ ou dans lequel la constante de proportionnalité est comprise notamment entre $1,2$ mGy·HU$^p$ et $1,2$ mGy·HU$^p$.

6. Procédé suivant l'une des revendications précédentes, dans lequel le niveau de bruit est une fonction linéaire par morceau du diamètre équivalent d'eau.

7. Procédé suivant la revendication 6, dans lequel le niveau de bruit est une fonction continue, définie par partie, du diamètre équivalent d'eau, en ayant exactement deux parties, la première partie étant une fonction constante, tandis que la deuxième partie est une fonction linéaire du diamètre équivalent d'eau.

8. Procédé suivant l'une des revendications précédentes, comprenant, en outre, le stade de procédé suivant :

   - troisième détermination (DET-3) d'une évaluation de dose spécifique en grandeur reposant sur le paramètre de dose de référence au moyen de l'unité (402) de calcul.

9. Procédé suivant l'une des revendications précédentes, comprenant, en outre le stade de procédé suivant :

   - quatrième détermination (DET-4) d'un paramètre d'enregistrement d'un appareil (420) de tomographie par ordinateur, reposant sur le paramètre de dose de référence au moyen de l'unité (402) de calcul, dans lequel une deuxième dose de rayons X correspond au paramètre de dose de référence, la deuxième dose de rayons X aurait, à l'enregistrement d'un deuxième jeu de données d'image CT, été absorbée à l'enregistrement d'un deuxième jeu de données d'image CT, dans le plan de coupe, si le deuxième jeu de données d'image CT avait été enregistré en utilisant le paramètre d'enregistrement au moyen de l'appareil (420) de tomographie par ordinateur.

10. Procédé suivant l'une des revendications 1 à 8, comprenant, en outre, les stades de procédé suivants :

    - deuxième réception (REC-2) d'un paramètre de dose réel d'un enregistrement d'un troisième jeu de données d'image CT du volume à étudier au moyen de l'interface (401), dans lequel le paramètre de dose réel correspond à la troisième dose de rayons X absorbée dans le plan de coupe à l'enregistrement du troisième jeu de données d'image CT, - correction (COR) de la puissance et/ou de la constante de proportionnalité, sur la base d'une comparaison entre le paramètre de dose de référence et le paramètre de dose réel au moyen de l'unité (402) de calcul.

11. Unité (400) de détermination de paramètres, comprenant les unités suivantes :

    - interface (401), constituée pour la première réception (REC1) d'un diamètre équivalent d'eau d'un plan de coupe d'un volume à étudier, - unité (402) de calcul, constituée pour la première détermination (DET-1) d'un niveau de bruit reposant sur le diamètre équivalent d'eau, le niveau de bruit étant une valeur de seuil supérieure du bruit d'un jeu de données d'image CT, constituée, en outre, pour la deuxième détermination (DET-2) d'un paramètre de dose de référence reposant sur le niveau de bruit et le diamètre équivalent d'eau, le paramètre de dose de référence correspondant à une première dose de rayons X, qui aurait, lors d'un enregistrement d'un premier jeu de données d'image CT du volume à étudier, été absorbée dans le plan de coupe, si le bruit du premier jeu d'image CT correspond au niveau de bruit, le paramètre de dose de référence étant inversement proportionnel à une puissance du niveau de bruit.

12. Unité (400) de détermination de paramètres, constituée, en outre, pour effectuer un procédé suivant l'une des revendications 2 à 10.

13. Appareil (420) de tomographie par ordinateur, comprenant une unité (400) de détermination de paramètres suivant la revendication 11 ou 12.

14. Produit de programme d'ordinateur, ayant un programme d'ordinateur, qui peut être chargé directement dans une mémoire (403) d'une unité (400) de détermination de paramètres, ayant des parties de programme pour effectuer tous les stades du procédé suivant l'une des revendications 1 à 10 lorsque les parties de programme sont exécutées par l'unité (400) de détermination de paramètres.

15. Support de mémoire déchiffrable par ordinateur, sur lequel des parties de programme déchiffrables et réalisables par une unité (400) de détermination de paramètres sont mises en mémoire pour effectuer tous les stades du procédé suivant l'une des revendications 1 à 10 lorsque les parties de programme sont réalisées par l'unité (400) de détermination de paramètres.

FIG 1

REC-1

DET-1

DET-2

DET-3

FIG 2

REC-1

DET-1

DET-2

DET-4

FIG 3

REC-1

DET-1

DET-2

COR

REC-2

DET-5

## FIG 4

## FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013103790 A